# EUROPEAN PATENT SPECIFICATION

(11) **EP 3 995 414 B1**
(45) Date of publication and mention of the grant of the patent: **23.04.2025**
(21) Application number: 20206579.3
(22) Date of filing: 10.11.2020
(51) Int. Cl.: B65G 1/04, B65G 1/14, A01K 67/033

(54) **STORAGE SYSTEM FOR STORING CRATES AND REARING UNIT FOR REARING OF INSECTS OR INSECT LARVAE OR WORMS**
LAGERSYSTEM ZUR LAGERUNG VON KÄSTEN UND AUFZUCHTEINHEIT ZUR AUFZUCHT VON INSEKTEN ODER INSEKTENLARVEN ODER WÜRMERN
SYSTEME DE STOCKAGE POUR LE STOCKAGE DE CASIERS ET UNITE D'ELEVAGE POUR L'ELEVAGE D'INSECTES OU DE LARVES D'INSECTES OU DE VERS

(43) Date of publication of application: 11.05.2022
(73) Proprietor: Bühler AG, 9240 Uzwil (CH)
(72) Inventor: GILLIS, Jacobus Henricus Antonius Maria, 4835 BG Breda (NL); DE GELDER, Vincent, 4207 VC Gorimchem (NL); HEIJNEN, Hendrikus Petrus, 5632 LB Eindhoven (NL); JANSEN, Maurits Petrus Maria, 4854 CA Bavel (NL)
(74) Representative: Vossius & Partner Patentanwälte Rechtsanwälte mbB

(56) References cited:
- WO-A1-2020/246878
- WO-A1-2022/055346
- DE-U1- 9 319 992
- JP-A- S58 104 804
- JP-U- S5 136 887
- JP-U- S5 248 287
- JP-U- S5 285 290
- KR-B1- 101 733 544
- NL-A- 9 300 418
- US-A- 3 253 416
- US-A1- 2015 225 187

## Description

The present disclosure relates to a storage system for storing crates according to claim 1 and to a rearing unit for rearing of insects or insect larvae or worms according to claim 13.

In the field of insect or worm rearing, crates are utilised which are specifically designed to house the insects or worms and a nutrient medium for the insect larvae or worms. In order to save space, the crates are stacked on each other to a certain height, and stored in a room having a condition climate adapted to the requirements of the insects or worms in order to provide suitable conditions for growth of the insects or worms. In general, multiple lanes of stacked crates are stored next to each other. The stacks of crates in the rearing room have to be supported by support elements, e.g. in form of pallets or rack systems as shown by US 10,405,528 B2. The stacked crates have to be stacked in such a way that they may be automatically picked up or moved, e. g., for control or further processing purposes such as checking growth and vital parameters or further processing of the insects or worms. This is usually done using a conveyor system, e. g., on rails. DE 93 19 992 U1 discloses a guide system for pallets as well as a transport device suitable for said guide system. KR 101 733 544 B1 relates to an automated storage system comprising an emergency unloading of cargo. WO 2020/246878 A1 is a document under Art. 54(3) EPC and relates to a system and method for rearing invertebrates.

It may occur that larvae or worms exit the crates which may lead to a cross-contamination of different lanes. Also, debris from the storage rooms may contaminate the floor. Currently, free standing rails are used which are difficult to clean because the washing fluid e.g. water spreads over the entire floor since the water is not effectively channelled.

The object of the present invention is therefore to avoid at least partly the disadvantages of the prior art and especially to provide a storage system for storing crates which prevents cross-contamination and may be easily cleaned. Furthermore, the support elements of the storage system shall be long-lasting and configured to allow storage and transport of the crates. Further objects of the present invention are also to provide a rearing unit for rearing of insects or insect larvae or worms.

Furthermore, contamination of adjacent lanes of stacked crates should be prevented and larvae which escaped from the crates or any fluid used in cleaning should not move across the lanes. It is also desirable that the lanes may be easily cleaned when there are no crates placed upon them.

These objects may be achieved by a support element, a support system and/or a storage system and/or a rearing unit as disclosed herein. The invention is defined in the independent claims. Dependent claims describe preferred embodiments.

In particular, a support element according to the present disclosure comprising an elongate element having a cross section that is substantially formed in an U-shape, comprising a bottom section and two opposite side walls extending from the bottom section, wherein the side walls each comprise a top portion, forming two elongate supporting areas, whereby the elongate supporting areas are configured to support a crate or a column of stacked crates, wherein inner side sections of the side walls each comprise a step preferably symmetrically protruding to the inside of the U-shape forming two elongate running surfaces, whereby the elongate running surfaces are configured to support a conveyor system for moving the crate or stacked crates.

Such a support element provides a stable support for the crates, also for stacked crates and at the same time are easily cleanable. The side walls extend from the same side of the bottom section for forming the U-shape together with the bottom section.

Alternatively, the support element is substantially formed from two L-shaped elements, each comprising a bottom section and a side walls extending from this bottom section. The two L-shaped elements form together a support element that is substantially formed in a U-shape, but comprising a discontinuous (not continuous) bottom section. The contact surface of the bottom sections between the two L-shaped elements is preferably sealed, at least partly.

Various embodiments may preferably implement the following features:
Preferred the support element is formed of concrete and therefore easily producible.

Preferably the concrete is cast on metal rails that the support element is more stable and resistant also against tension.

Preferred the running surfaces of the support element are covered with a steel coating to provide a perfect surface for the conveyor system. Preferably, the running surfaces of the support element are covered with a stainless steel coating, so that the steel coating does not rust, also in a potentially aggressive environment. E. g., the coating comprising a band material which preferably is cast-in during fabrication of the support element. Such running surfaces are advantageously, especially if the conveyor system comprising wheels or other rotational means, because they create guides and long-lasting surfaces.

Alternatively, the support element is formed of steel which allows to provide plane or even surfaces. Preferably the support element is formed of stainless steel, so that the support element does not rust, also in a potentially aggressive environment.

Preferred the support element is monolithic that an easy handling of the support member is guaranteed.

Alternatively the support element is as multi-piece element. Thereby, especially the width between the support element pieces can be adjusted according on a respective size of the crate.

The present disclosure refers also to a support system for storing crates comprising at least one support element as aforementioned and a conveyor system, wherein the conveyor system extends in size for being moveable underneath the crates supported by the supporting element without interfering with the crates.

This support system provides the advantages of the support element as well as a compact and a space-saving solution. A correct handling of the crates and the columns of stacked crates is given.

The measurements at the height of the conveyor system are chosen so that - in an arranged state of the conveyor system - the upper edge of the conveyor system is lower than the top portion of the side walls

Preferably, the conveyor system is configured for being supported by the elongate running surfaces formed by the steps of the support element. More preferably, the conveyor system comprising wheels or other rotational means configured for being supported by the elongate running surfaces formed by the steps of the support element. Thereby, a reliable support system is provided.

Alternatively, the conveyor system is configured to be arranged on the floor or on the bottom section of the support element. This alternative solution is preferably used when the conveyor system comprising automated guided vehicles (AGV).

Preferred the conveyor system includes means for lifting the crates in order to move the crates or columns of stacked crates along the support element. Thereby, an easy handling of the crates is ensured.

Preferred the conveyor system is movable by a drive that the conveyor system can be moved inside of the support system.

Alternatively the conveyor system comprising a drive for moving the conveyor system along the elongation of the support element what facilitates the handling of the crates or of the columns of stacked crates.

Preferably, the conveyor system is, at least partly, autonomous moveable, whereby a respective control for controlling the conveyor system is provided.

The invention refers to a storage system for storing crates comprising a plurality of support element as aforementioned and a plurality of crates arranged on the top portions of the side walls of the support elements, wherein the crates have a substantially rectangular perimeter defined by upstanding front and rear walls and opposing side walls, wherein preferably the length of the crates between the front and rear walls substantially corresponds to the distance of the side walls of the support element. Alternatively the width of the crates between the both side walls substantially corresponds to the distance of the side walls of the support element. Preferably, at least one dimension of the crate fits with the respective dimension of the support element.

Alternatively or additionally the invention refers also to a storage system for storing crates comprising at least one support system as aforementioned and a plurality of crates arranged on the top portions of the side walls of the support element, wherein the crates have a substantially rectangular perimeter defined by upstanding front and rear walls and opposing side walls, wherein preferably the length of the crates between the front and rear walls substantially corresponds to the distance of the side walls of the support element. Alternatively the width of the crates between the both side walls substantially corresponds to the distance of the side walls of the support element. Preferably, at least one dimension of the crate fits with the respective dimension of the support element.

Such storage systems are easy to handle with the advantages as mentioned before.

Preferred the storage system the support elements are arranged consecutively in a longitudinal direction along the elongation of the support elements for a space-saving arrangement. The support elements are preferably sealed on their contact surfaces or between each other, respectively.

Preferably, the distance between at least two longitudinal spaced support elements is chosen so that provided air can circulate or pass between the crates for providing optimal conditions for the rearing. At least partly the distance between two support elements is chosen so that an operator can pass between the stacked crates, e.g., for a visual inspection.

Preferred the crates are stacked in at least one column of stacked crates what results in a space-saving arrangement.

Alternatively or additionally, the crates or columns of stacked crates are arranged adjacently on the at least one support element.

Preferred the storage system comprises a conveyor system for transportation of the crates, whereby the conveyor system is configured for being supported by the elongate running surfaces formed by the steps of the support element for moving the conveyor system along the elongation of the support element underneath the crates supported by the supporting element without interfering with the crates. The conveyor system preferably comprises wheels or other rotational means.

Preferred the conveyor system is positioned by an observation system, wherein the observation system comprising at least one laser or at least one camera. By the observation system the correct position of the conveyor system can be determined and/or controlled. This positioning of the conveyor system can be laser-based. If a camera as observation system is used, the position of the conveyor system is defined based on an analysis of the pictures made by the camera.

Preferred the crates are adapted to allow rearing of insects or insect larvae or worms. Preferably Black Soldier Flies (BSF) larvae are reared.

The invention refers also to a rearing unit for rearing of insects or insect larvae or worms comprising a rearing room and a storage system as aforementioned, whereby an easy handling of the crates especially during the rearing process of the insects or insect larvae or worms and the respective process steps is guaranteed.

The invention will be described with reference to the following drawings.
Fig. 1 shows a support element for storing crates of the storage system according to the present invention,
Fig. 2 shows the support element of Fig. 1 in use,
Fig. 3 shows a rearing unit with a storage system for storing crates according to the present invention,
Figs. 4a and 4b show each a support system for storing crates of the storage system according to the present invention,
Figs. 5a to 5e show alternative embodiments of a support element, and
Figs. 6a to 6c show an arrangement of a support element according to Fig. 5a in three steps.

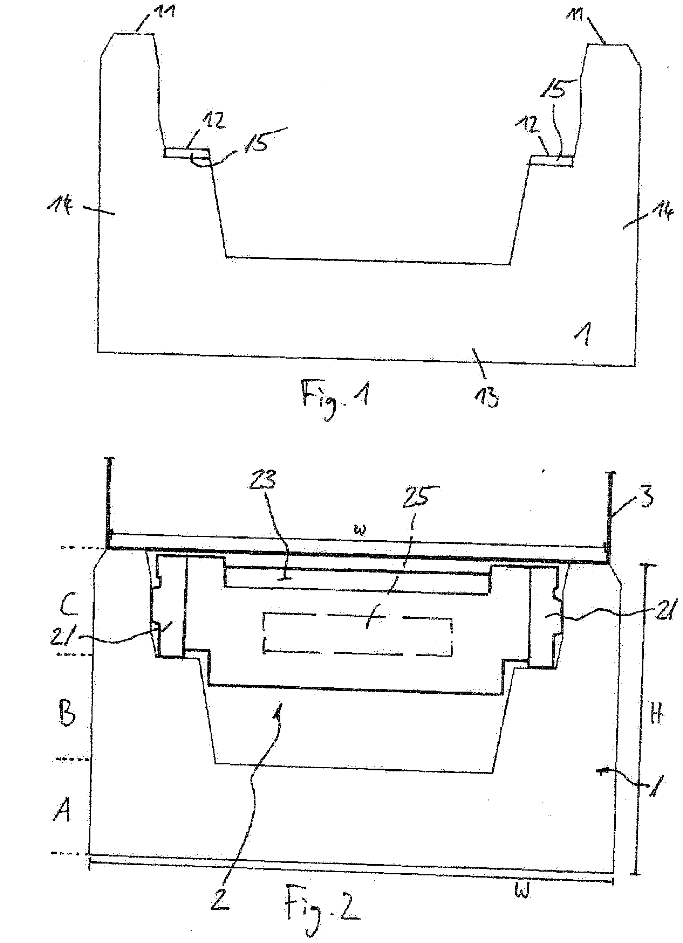

With the present disclosure, a support element 1 according to Fig. 1 is proposed. The support element 1 is formed of concrete and is monolithic. The support element 1 is an elongate element having a cross-section substantially formed in a U-shape. I. e., the support element 1 essentially forms a vertically aligned U and is extended in a horizontal direction. When viewed in a cross-section, the support element 1 comprises a continuous bottom section 13 and two opposite side walls 14 extending from the bottom section13. Preferably they are formed in a symmetrical manner.

The side walls 14 each comprise a top portion 11 which form two elongate supporting areas. The sidewalls each comprise a step 12 which symmetrically protrudes to the inside of the U-shape. Said steps 12 form two elongate running surfaces. Said running surfaces are aligned in the same direction as the supporting areas 11. The running surfaces of the support element 1 are covered with a steel coating 15 which is cast-in and preferably is a stainless steel coating.

Running surfaces are configured to support a conveyor system 2 for moving the crates 3. The conveyor system 2 may be a so-called satellite which is an autonomous robot having wheels 21 or other rotational means adapted to the width of the running surfaces and having lifting means 23 for carrying and transporting the crates 3. A drive 25 is provided for moving the conveyor system 2 along the supporting elements 1. This drive 25 is preferably designed for driving the conveyor system 2 and also for driving the lifting means 23.

The conveyor system 2 may thus drive below a single crate 3 or column 39 of stacked crates which are stored on the supporting elements 1 and lift them using the lifting means 23 until the crates 3 lose contact with top portion 11 of the supporting elements 1. The conveyor system 2 might lift several columns of stacked crates at the same time.

The conveyor system 2 may then move the crates 3 to another place in the rearing unit (rearing facility). The lifting means 23 may be designed as hydraulic, electrical or mechanical means or any other means suitable for lifting and carrying crate 3, e. g. as a scissor lift. The conveyor system 2 may additionally comprise side wheels to avoid direct contact of the conveyor system 2 with the inner side of the side walls 14 of the support elements 1, and thus avoid friction.

The support elements 1 may be connected to form a line or lane. They may be mounted on the ground or floor and may be sealed in respect to each other such that no fluid and larvae can enter a lane or a space under the lanes.

Shims may be used to achieve a desired height of the support elements 1, e. g. in case the floor is not level. In order to be cleanable the space between the support element 1 and the floor may be sealed.

Multiple lanes may be positioned adjacent to each other in a rearing (storage) room. The width between the lanes may be provided in such a manner that air can circulate between the crates or columns of staked crates. Preferably the width between the lanes is chosen that the staff may pass between the lanes and perform maintenance or inspection operations. The width may be between 200 and 1000 mm, preferably between 300 and 400 mm and more preferably 360 mm. The space between two spaced support elements or lanes of support elements is preferably chosen that escaping larvae or liquids are collected and cannot escape from the space between two lanes. The aforementioned space corresponds preferably to 20% up to 200% of the larvae stored in the crates of one lane. By this measures, cleaning is facilitated and spreading of larvae or matured insects throughout the whole system is avoid. By the U-shape as described above fluid and larvae may be prevented from crosscontaminating different lanes in the rearing room. In order to provide good cleanability of the insides of the U-shaped support elements 1, the surfaces preferably not comprise holes or grooves. I. e., the surface of the support elements 1 should be smooth. When viewed in a cross-section, each support element 1 thus has a closed circumference. This also allows that the support elements 1 may be easily cleaned by effective channelling of water.

According to the invention, the support elements 1 may be formed from concrete. It may also be advantageous to cast concrete on metal rails, in which case, however, concrete still forms the surface of the support elements 1. In addition to any of the materials, the running surfaces may comprise a coating made of steel. This may reduce the point load on the concrete and provide a perfect surface for the wheels 21 of the conveyor system 2. In another preferred embodiment the support elements 1 may be formed from stainless steel.

In order to prevent crates 3 from toppling and to provide easy access a transport means, the support elements 1 should meet certain tolerances to ensure that the lanes and tracks are straight and homogenous. The materials preferably used according to the present invention also avoid the problem of rust which occurs in the currently used systems. Due to the often humid, ammonia rich and warm climate inside a rearing room and the use of metal rails, rust may significantly decrease the lifetime of such a storage system 51.

In the case the support elements 1 are formed of concrete, they may be cast at the respective location. This saves costs for the reinforcement of concrete because the support elements 1 do not need to be transported. It has to be considered though, that the tolerances may be harder to meet when the support elements 1 are cast on location. Furthermore, the floor has to be prepared more thoroughly.

The functions and requirements of the support elements 1 are now described using an example. However, other parameters such as width, height, force, weight etc. may act or be required. The support elements 1 of Fig. 1 and Fig. 2 substantially correspond. The description of similar or the same parts may thus be omitted.

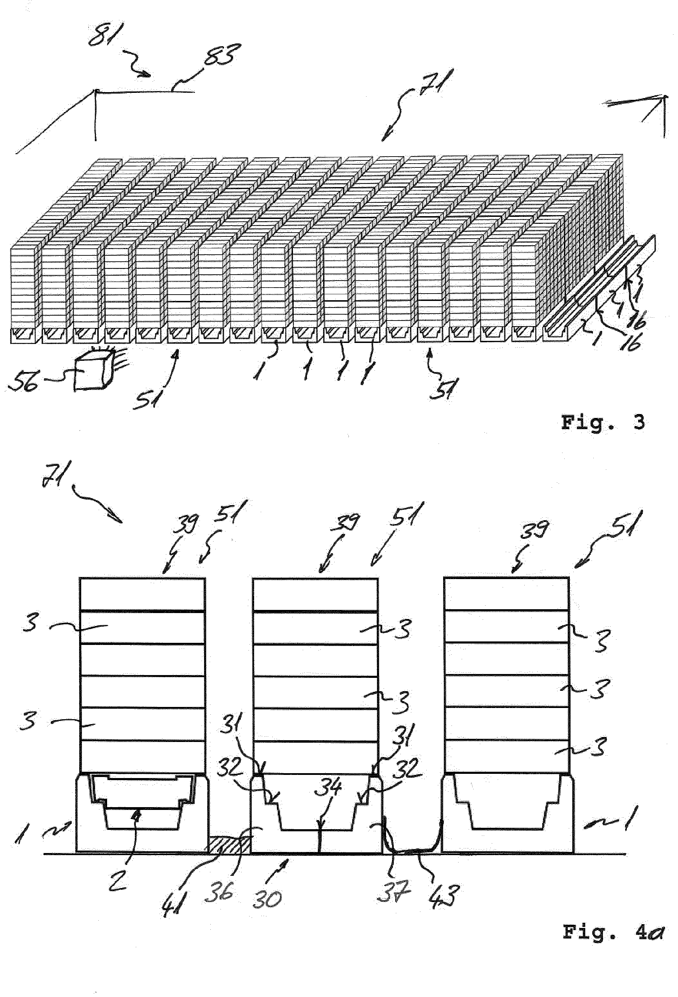

According to an exemplary configuration as shown by Fig. 3, the support elements 1 have to support stacks of up to twelve crates 3. Each crate 3 may have a weight of 40 kg. The crates 3 may be rectangular having a shorter side and a longer side. The longer side (width) w may be between 400 and 1000 mm, preferably 800 mm long. The shorter side (length) may be between 200 and 800 mm, preferably 600 mm long.

Each support element 1 may have height H of 100 to 600 mm, in the present exemplary embodiment 490 mm. The height is also defined by the required space between two lanes of support element for receiving at least 50% up to 100% of the reared larvae in one lane. The lower the crates 3 are stacked or the smaller the crates are the lower the height H of the support elements 1 can be chosen.

The width W of the support elements 1 may substantially correspond to the width w of the crates 3.

For the sake of stability, the support elements 1 may be formed wider than the crates 3. When viewed in a cross-section, the U-shaped support elements 1 may be divided in at least two sections. In the present example as shown in Fig. 2, the support element 1 may be described in three sections. The three sections may roughly be of the same height and thus substantially divide the U-shape in thirds. In the present example, the bottom section A between the floor of the storage room and the bottom part 13 of the U-shape is 150 mm high. On the bottom section A, potential escaped larvae, debris, residuals and fluid may be collected. The middle section B is formed between the bottom section 13 of the U-shape and the step 12 which is symmetrically formed on the side walls 14 of the U-shape. The step 12 thereby forms the running surface. Preferably, the side walls 14 of the middle section B connecting the bottom section 13 and the step 12 are not exactly vertical. Preferably the side walls 14 of the middle section B are inclined in at least one segment. This allows for effectively collecting residuals, larvae and fluids and facilitates cleaning. In the present example, both side walls 14 are formed at an angle of 20° with respect to each other and are 169 mm high. The top section C is formed between the step 12 or running surface, respectively, and the supporting area, i. e. the top portion 11. In the present example, the top section C is 171 mm high. The top portion 11 preferably is bevelled downwards on at least one side. The inner side of the side walls 14 of the top section C forming the top portion 11 are preferably inclined in at least one section, i. e. not exactly vertical over the entire length.

The supporting areas (top portion 11) may be between 50 and 100 mm wide each. In the present example, both supporting areas are around 70 mm wide each, leading to a distance of the inner side of the side walls 14 of the top section of 655 mm, less the bevelled portion. The running surface supporting the conveyor system 2 in the present example is 70 mm wide on both sides.

The conveyor system 2 extends in size for being moveable underneath the crates 3 supported by the supporting elements 1 without interfering with the crates 3. The conveyor system 2 or satellite, respectively, may carry up to 4 columns of stacked crates 3 at the same time. The weight of the conveyor system 2 itself of ca. 400 kg must also be taken into account.

An observation system 56 (see Fig. 3) is provided for positioning of the conveyor system 2 inside of the support element 1. The observation system 56 comprises one or more laser or one or more cameras.

In order to be cleanable, larvae or any fluid should not be able to move across the lanes of support elements 1. At least during production or rearing, there may be at least one empty lane which may be cleaned before new crates 3 are placed on it. During cleaning, the cleaning water carrying larvae, fluid or residuals should not contaminate the adjacent lane or under the lanes. This is achieved by the structure of the support elements 1 as presented above.

In summary, the present disclosure provides a support element 1 which may be used in an insect rearing unit (facility). Said support element 1 may be provided in multiple and connected to form lanes or lines. Thus, a modular and expandable system may be formed. Due to the simplified and robust construction it may have a lifetime of 20 years and more without deformation or impact of shear stress. Also, rust may be prevented since materials such as concrete and stainless steel are utilised. Furthermore, a conveyor system 2 for conveying the crates 3 may be supported by and fit into the support elements 1. By having a closed structure substantially without holes and grooves, cleaning of the rearing room is facilitated and cross-contamination between different lanes may be prevented. Cleaning water may be effectively channelled along the lanes.

Figure 3 shows a rearing unit 81 for rearing of insects or insect larvae or worms comprising a rearing room 83 (schematically shown) and a storage system 71.

The storage system 71 for storing crates 3 comprises support elements 1 or support systems 51, respectively, and a plurality of crates 3 arranged on the top portions 11 of the side walls 14 of the support elements 1.

The crates 3 are stacked in columns 39 of stacked crates 3 and are arranged adjacently on the support elements 1. The crates 3 have a substantially rectangular perimeter defined by upstanding front and rear walls and opposing side walls, wherein preferably the length of the crates 3 between the front and rear walls substantially corresponds to the distance between the side walls 14 of the support elements 1.

The support elements 1 are arranged in rows, consecutively in a longitudinal direction along the elongation of the support elements 1. The space 16 between multiple support elements 1 arranged in line to form lanes are preferably sealed.

In reference to Fig. 4a showing the storage system 71, the support system 51 for storing crates 3 comprises a plurality of support elements 1 und 30 which are arranged parallel to each other. Furthermore, the support system 51 comprises a conveyor system 2. Preferably each lane of support elements 1 or 30 is provided with one conveyor system 2. Alternatively, the conveyor system 2 can switch from one lane to another lane.

The bottom of the space between two support elements 1 may be provided with a sealing. E. g., the sealing 41 comprises concrete, preferably flow mortar or sealants. Alternatively or additionally, the sealing 43 comprising a foil or cover, e. g., made of plastic, which is glued on.

The U-shaped support elements 30 (see example in Fig. 4a) described above may also be implemented by connecting two mirrored L-shaped elements 36 and 37 comprising the step 32, i. e. running surface, as well as the top portion 31 as supporting area as set forth above. The contact area 34 of the two L-shaped elements 36 and 37 is sealed.

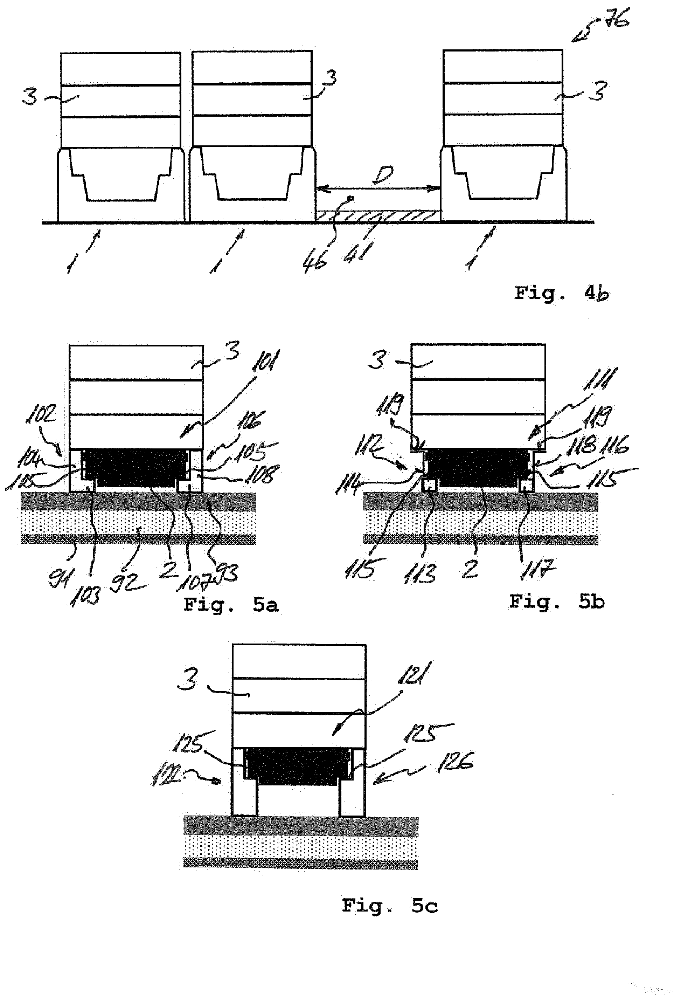

In the storage system 76 according to Fig. 4b two support elements 1 are arranged directly to each other in longitudinal direction and another support element 1 is arranged spaced from the support element 1 nearby in a width D. The width D between the two support elements 1 which are spaced from each other in longitudinal direction may be between 200 and 1000 mm, preferably between 300 and 400 mm and more preferably 360 mm. The space 46 between the two spaced support elements 1 or lanes of support elements 1 is preferably chosen that escaping larvae or liquids are collected and cannot escape from the space between two lanes. The aforementioned space corresponds preferably to 20% up to 200% of the larvae stored in the crates of one lane. By this measure, cleaning is facilitated and spreading of larvae or matured insects throughout the whole system is avoided.

Figs. 5a to 5e show each different alternative embodiments of the inventive support element. For levelling the existing floor 91 an insulation foam layer 92 may be provided which may be covered by a concrete layer 93. Alternatively, for levelling the existing floor 91 the concrete layer 93 may also be provided directly on the existing floor 91.

The support element 101 (see Fig. 5a) comprising two L-shaped elements 102 and 106 made from concrete. The L-shaped elements 102 and 106 are arranged in a respective distance to each other depending on the size of the crates 3 arranged thereon.

The L-shaped element 102 comprising a bottom section 103 and a side wall 104. The running surface 105 is formed by the upper surface of the bottom section 103. The L-shaped element 106 comprising a bottom section 107 and a side wall 108. The other running surface 105 is formed by the upper surface of the bottom section 107.

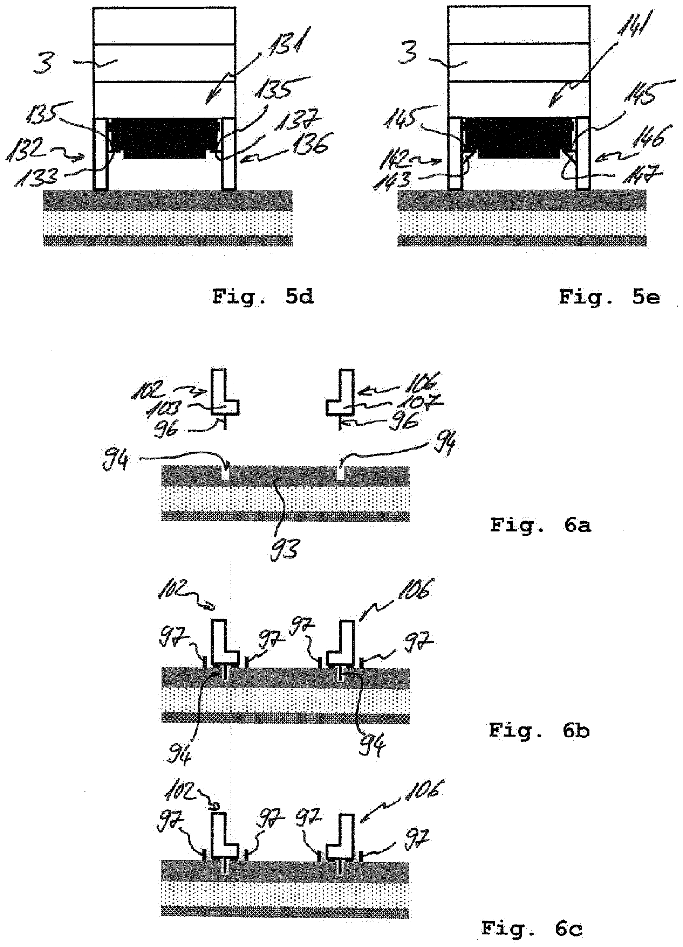

For arrangement of the support element 101 openings 94 are provided in the concrete layer 93 for receiving anchorage elements 96 extending from the bottom section 103 or 106 of the elements 112 and 116 (Fig. 6a). The anchorage elements 96 are made of metal and may be casted-in when the elements 112 and 116 are casted. The anchorage elements 96 may have a longitudinal shape (then the openings 94 are preferably formed as slits) or are bar-shaped (then the openings 94 are preferably formed as holes).

The openings 94 are filled with a gluing compound, e. g. a synthetic glue or flow mortar, before the elements 112 and 116 are mounted (Fig. 6b). For supporting the elements 112 and 116 against shearing forces formwork elements 97 are provided on both sides of the elements 112 and 116 which run in longitudinal direction.

The space between the formwork elements 97 and the respective elements 112 or 116 is then filled with a gluing compound, e. g. a synthetic glue or flow mortar (Fig. 6c).

The support element 111 (see Fig. 5b) comprising two Z-shaped elements 112 and 116 made from metal, preferably made from stainless steel. The Z-shaped elements 112 and 116 are arranged in a respective distance to each other depending on the size of the crates 3 arranged thereon.

The Z-shaped element 112 comprising a box-shaped bottom section 113 and a side wall 114. The running surface 115 is formed by the upper surface of the bottom section 113. A horizontal section 119 extending from the upper end of the side wall 114 for forming a top portion by which the crates 3 are supported. The Z-shaped element 116 comprising a box-shaped bottom section 117 and a side wall 118. The other running surface 115 is formed by the upper surface of the bottom section 117. A horizontal section 119 extending from the upper end of the side wall 118 for forming a top portion by which the crates 3 are supported.

The support element 121 (see Fig. 5c) comprising two elements 122 and 126 made from concrete. The elements 122 and 126 are arranged in a respective distance to each other depending on the size of the crates 3 arranged thereon.

The element 122 comprising a stepped section which forms the running surface 125. The element 126 comprising a stepped section which forms the other running surface 125.

The support element 131 (see Fig. 5d) comprising two elements 132 and 136 made from concrete. The elements 132 and 136 are arranged in a respective distance to each other depending on the size of the crates 3 arranged thereon.

The element 132 comprising a flat extension 133 which forms the running surface 135. The element 136 comprising a flat extension 137 which forms the other running surface 135. The extensions 133 and 137 are made of metal, preferably made of stainless steel.

The support element 141 (see Fig. 5e) comprising two elements 142 and 146 made from concrete. The elements 142 and 146 are arranged in a respective distance to each other depending on the size of the crates 3 arranged thereon.

The element 142 comprising a console 143 which forms the running surface 145. The element 146 comprising a console 147 which forms the other running surface 145. The consoles 143 and 147 may be made of metal, preferably made of stainless steel, or may be made of concrete, preferably formed when the element 142 or 146 is casted.

Other aspects, features, and advantages will be apparent from the summary above, as well as from the description that follows, including the figures and the claims.

While the invention has been illustrated and described in detail in the drawings and foregoing description, such illustration and description are to be considered illustrative or exemplary and not restrictive. It will be understood that changes and modifications may be made by those of ordinary skill within the scope of the following claims.

Furthermore, in the claims the word "comprising" does not exclude other elements or steps, and the indefinite article "a" or "an" does not exclude a plurality. A single unit may fulfil the functions of several features recited in the claims. The terms "essentially", "about", "approximately" and the like in connection with an attribute or a value particularly also define exactly the attribute or exactly the value, respectively. Any reference signs in the claims should not be construed as limiting the scope.

### List of reference signs

- 1: Support element
- 11: Top portion
- 12: Step
- 13: Bottom part
- 14: Side wall
- 15: Steel coating
- 16: Space between support elements in longitudinal direction
- 2: Conveyor system
- 21: Wheel
- 23: Lifting means
- 25: Drive
- 3: Crate
- 30: Support element
- 31: Top portion
- 32: Step
- 34: Contact area
- 36: L-shape part
- 37: L-shape part
- 39: Column of stacked crates 3
- 41: Sealing
- 43: Foil
- 46: Space between spaced support elements
- 51: Support system
- 56: Observation means
- 71: Storage system
- 76: Storage system
- 81: Rearing unit
- 83: Rearing room
- 91: Existing floor
- 92: Insulation foam layer
- 93: Concreate layer
- 94: Opening in 93
- 96: Anchorage element
- 97: Formwork element
- 101: Support element
- 102: L-shaped element
- 103: Bottom section
- 104: Side wall
- 105: Running surface
- 106: L-shaped element
- 107: Bottom section
- 108: Side wall
- 111: Support element
- 112: L-shaped element
- 113: Bottom section
- 114: Side wall
- 115: Running surface
- 116: L-shaped element
- 117: Bottom section
- 118: Side wall
- 119: Horizontal section
- 121: Support element
- 122: Element
- 125: Running surface
- 126: Element
- 131: Support element
- 132: Element
- 133: Flat extension
- 135: Running surface
- 136: Element
- 137: Flat extension
- 141: Support element
- 142: Element
- 143: Console
- 145: Running surface
- 146: Element
- 147: Console
- w: Width of the crate
- A: Bottom section
- B: Middle section
- C: Top section
- D: Width between two support elements
- H: Height of the support element
- W: Width of the support Element

## Claims

1. Storage system for storing crates comprising:
a plurality of support elements (1, 30; 101; 111; 121; 131; 141) for storing crates (3), wherein
the support elements (1, 30; 101; 111; 121; 131; 141) are elongate elements having a cross section that is substantially formed in an U-shape, comprising a continuous bottom section (13; 103; 113) and two opposite side walls (14; 104; 114) extending from the bottom section (13; 103; 113),
wherein the side walls (14) each comprise a top portion (11), forming two elongate supporting areas, whereby the elongate supporting areas are configured to support a crate (3) or a column (39) of stacked crates (3),
wherein inner side sections of the side walls (14; 104; 114) each comprise a step (12) protruding, preferably symmetrically, to the inside of the U-shape forming two elongate running surfaces, whereby the elongate running surfaces are configured to support a conveyor system (2) for transporting the crate (3);
wherein each of the plurality of support elements (1) is monolithic, and
wherein each of the plurality of support elements (1, 30; 101; 121; 131; 141) is formed of concrete or steel;
wherein the storage system further comprises:
a plurality of crates (3) arranged on the top portions of the side walls (14; 104; 114; 124; 134; 144) of the support elements (1, 30; 101; 111; 121; 131; 141),
wherein the crates (3) have a substantially rectangular perimeter defined by upstanding front and rear walls and opposing side walls, wherein preferably the length of the crates (3) between the front and rear walls substantially corresponds to the distance between the side walls (14; 104; 114; 124; 134; 144) of the support element (1; 101; 111; 121; 131; 141).

2. Storage system according to claim 1, wherein
the concrete is cast on metal rails.

3. Storage system according to claim 1 or 2, wherein the running surfaces of each of the plurality of the support elements are covered with a steel coating (15), preferably with a stainless steel coating.

4. Storage system according to claim 1, wherein each of the plurality of support elements (111) is formed of stainless steel.

5. Storage system according to any one of the preceding claims, further comprising:
a conveyor system (2), wherein
preferably the conveyor system (2) is configured for being supported by the elongate running surfaces (105; 115; 125; 135; 145) formed by the steps (12) of the support elements (1, 30; 101; 111; 121; 131; 141) and wherein
the conveyor system (2) extends in size for being moveable underneath the crates (3) supported by the supporting elements (1, 30; 101; 111; 121; 131; 141) without interfering with the crates (3).

6. Storage system according to claim 5, wherein the conveyor system (2) includes means for transporting, preferably for lifting, the crates (3) in order to move the crates (3) or a column (39) of stacked crates (3) along the support elements (1, 30; 101; 111; 121; 131; 141).

7. Storage system according to claim 5 or 6, wherein the conveyor system (2) is movable by a drive (25) or
further comprises a drive (25) for moving the conveyor system (2) along the elongation of the support elements (1, 30).

8. Storage system according to any one of the preceding claims, the storage system (51) comprising at least two support elements (1, 30), arranged consecutively in a longitudinal direction along the elongation of the support elements (1, 30).

9. Storage system according to any one of the preceding claims, wherein the crates (3) are stacked in at least one column (39) of stacked crates (3) and/or
wherein the crates (3) or columns (39) of stacked crates (3) are arranged adjacently on the at least one support element (1, 30).

10. Storage system according to any one of claims 5 to 9, insofar as being dependent on claim 5,
the conveyor system (2) comprising wheels (21) or other rotational means.

11. Storage system according to claim 10, wherein the conveyor system (2) is positioned by an observation system (56),
the observation system (56) comprising at least one laser and/or at least one camera.

12. Storage system according to any one of the preceding claims, wherein the crates (3) are adapted to allow rearing of insects or insect larvae or worms.

13. Rearing unit (81) for rearing of insects or insect larvae or worms comprising a rearing room (83) and a storage system (71; 76) according to any one of claims 1 to 12.

## Patentansprüche

1. Lagersystem zur Lagerung von Kästen, aufweisend:
mehrere Trägerelemente (1, 30; 101; 111; 121; 131; 141) zur Lagerung von Kästen (3), wobei
die Trägerelemente (1, 30; 101; 111; 121; 131; 141) längliche Elemente mit einem Querschnitt sind, der im Wesentlichen U-förmig ausgebildet ist, die einen durchgehenden Bodenabschnitt (13; 103; 113) und zwei gegenüberliegende Seitenwände (14; 104; 114) aufweisen, die sich ausgehend von dem Bodenabschnitt (13; 103; 113) erstrecken,
wobei die Seitenwände (14) jeweils einen oberen Abschnitt (11) aufweisen, der zwei längliche Abstützbereiche bildet, wobei die länglichen Abstützbereiche dazu ausgelegt sind, einen Kasten (3) oder eine Säule (39) aus gestapelten Kästen (3) abzustützen,
wobei innere Seitenabschnitte der Seitenwände (14; 104; 114) jeweils eine Stufe (12) aufweisen, die, vorzugsweise symmetrisch, zur Innenseite der U-Form vorsteht, um zwei längliche Laufflächen zu bilden, wobei die länglichen Laufflächen dazu ausgelegt sind, ein Fördersystem (2) zum Transportieren des Kastens (3) abzustützen;
wobei jedes mehreren Trägerelemente (1) monolithisch ausgebildet ist, und
wobei jedes mehreren Trägerelemente (1, 30; 101; 121; 131; 141) aus Beton oder Stahl gebildet ist;
wobei das Lagersystem ferner aufweist:
mehrere Kästen (3), die an den oberen Abschnitten der Seitenwände (14; 104; 114; 124; 134; 144) der Trägerelemente (1, 30; 101; 111; 121; 131; 141) angeordnet sind,
wobei die Kästen (3) einen im Wesentlichen rechteckförmigen Umfang haben, der durch eine aufrechte Vorder- und Rückwand und gegenüberliegende Seitenwände definiert ist, wobei vorzugsweise die Länge der Kästen (3) zwischen der Vorder- und Rückwand im Wesentlichen dem Abstand zwischen den Seitenwänden (14; 104; 114; 124; 134; 144) des Trägerelements (1; 101; 111; 121; 131; 141) entspricht.

2. Lagersystem nach Anspruch 1, wobei der Beton auf Metallschienen gegossen ist.

3. Lagersystem nach Anspruch 1 oder 2, wobei die Laufflächen jedes der mehreren Trägerelemente mit einer Stahlbeschichtung (15), vorzugsweise mit einer Edelstahlbeschichtung, versehen sind.

4. Lagersystem nach Anspruch 1, wobei jedes der mehreren Trägerelemente (111) aus Edelstahl gebildet ist.

5. Lagersystem nach einem der vorhergehenden Ansprüche, ferner aufweisend:
ein Fördersystem (2), wobei das Fördersystem (2) vorzugsweise dazu ausgelegt ist, von den länglichen Laufflächen (105; 115; 125; 135; 145), die aus den Stufen (12) der Trägerelemente (1, 30; 101; 111; 121; 131; 141) gebildet sind, abgestützt zu werden, und wobei das Fördersystem (2) sich in der Größe so erstreckt, dass es unter den von den Trägerelementen (1, 30; 101; 111; 121; 131; 141) abgestützten Kästen (3) bewegt werden kann, ohne die Kästen (3) zu beeinträchtigen.

6. Lagersystem nach Anspruch 5, wobei das Fördersystem (2) Mittel zum Transportieren, vorzugsweise zum Anheben, der Kästen (3) aufweist, um die Kästen (3) oder eine Säule (39) aus gestapelten Kästen (3) entlang der Trägerelemente (1, 30; 101; 111; 121; 131; 141) zu bewegen.

7. Lagersystem nach Anspruch 5 oder 6, wobei das Fördersystem (2) durch einen Antrieb (25) bewegbar ist oder ferner einen Antrieb (25) zum Bewegen des Fördersystems (2) entlang der Erstreckung der Trägerelemente (1, 30) aufweist.

8. Lagersystem nach einem der vorhergehenden Ansprüche, wobei das Lagersystem (51) zumindest zwei Trägerelemente (1, 30) aufweist, die in Längsrichtung entlang der Erstreckung der Trägerelemente (1, 30) aufeinanderfolgend angeordnet sind.

9. Lagersystem nach einem der vorhergehenden Ansprüche, wobei die Kästen (3) in zumindest einer Säule (39) aus gestapelten Kästen (3) gestapelt sind und/oder
wobei die Kästen (3) oder Säulen (39) aus gestapelten Kästen (3) nebeneinander auf dem zumindest einen Trägerelement (1, 30) angeordnet sind.

10. Lagersystem nach einem der Ansprüche 5 bis 9, soweit von Anspruch 5 abhängig, wobei das Fördersystem (2) Räder (21) oder andere Rotationsmittel aufweist.

11. Lagersystem nach Anspruch 10, wobei das Fördersystem (2) über ein Beobachtungssystem (56) positioniert wird, wobei das Beobachtungssystem (56) zumindest einen Laser und/oder zumindest eine Kamera aufweist.

12. Lagersystem nach einem der vorhergehenden Ansprüche, wobei die Kästen (3) zur Aufzucht von Insekten oder Insektenlarven oder Würmern eingerichtet sind.

13. Aufzuchteinheit (81) zur Aufzucht von Insekten oder Insektenlarven oder Würmern mit einem Aufzuchtraum (83) und einem Lagersystem (71; 76) nach einem der Ansprüche 1 bis 12.

## Revendications

1. Système de stockage pour le stockage de caisses, comprenant :
une pluralité d'éléments de support (1, 30 ; 101 ; 111 ; 121 ; 131 ; 141) destinés à stocker des caisses (3), dans lequel
les éléments de support (1, 30 ; 101 ; 111 ; 121 ; 131 ; 141) sont des éléments allongés présentant une section transversale qui est sensiblement en forme de U, comprenant une section inférieure continue (13 ; 103 ; 113) et deux parois latérales opposées (14 ; 104 ; 114) s'étendant depuis la section inférieure (13 ; 103 ; 113),
dans lequel les parois latérales (14) comprennent chacune une partie supérieure (11), formant deux zones de support allongées, où les zones de support allongées sont configurées pour supporter une caisse (3) ou une colonne (39) de caisses empilées (3),
dans lequel des sections latérales intérieures des parois latérales (14 ; 104 ; 114) comprennent chacune un palier (12) faisant saillie, de préférence symétriquement, vers l'intérieur de la forme en U formant deux surfaces de roulement allongées, où les surfaces de roulement allongées sont configurées pour supporter un système convoyeur (2) pour transporter la caisse (3) ;
dans lequel chacun de la pluralité d'éléments de support (1) est monobloc, et
dans lequel chacun de la pluralité d'éléments de support (1, 30 ; 101 ; 121 ; 131 ; 141) est formé de béton ou d'acier ;
dans lequel le système de stockage comprend en outre :
une pluralité de caisses (3) disposées sur les parties supérieures des parois latérales (14 ; 104 ; 114 ; 124 ; 134 ; 144) des éléments de support (1, 30 ; 101 ; 111 ; 121 ; 131 ; 141),
dans lequel les caisses (3) présentent un périmètre sensiblement rectangulaire défini par des parois avant et arrière verticales et des parois latérales opposées, dans lequel de préférence la longueur des caisses (3) entre les parois avant et arrière correspond sensiblement à la distance entre les parois latérales (14 ; 104 ; 114 ; 124 ; 134 ; 144) de l'élément de support (1 ; 101 ; 111 ; 121 ; 131 ; 141).

2. Système de stockage selon la revendication 1, dans lequel le béton est coulé sur des rails métalliques.

3. Système de stockage selon la revendication 1 ou 2, dans lequel les surfaces de roulement de chacun de la pluralité des éléments de support sont recouvertes d'un revêtement en acier (15), de préférence d'un revêtement en acier inoxydable.

4. Système de stockage selon la revendication 1, dans lequel chacun de la pluralité des éléments de support (111) est formé d'acier inoxydable.

5. Système de stockage selon l'une quelconque des revendications précédentes, comprenant en outre :
un système convoyeur (2), dans lequel
de préférence le système convoyeur (2) est configuré pour être supporté par les surfaces de roulement allongées (105 ; 115 ; 125 ; 135 ; 145) formées par les paliers (12) des éléments de support (1 ; 30 ; 101 ; 111 ; 121 ; 131 ; 141) et dans lequel
le système convoyeur (2) s'étend en taille de manière à pouvoir être déplacé sous les caisses (3) supportées par les éléments de support (1, 30 ; 101 ; 111 ; 121 ; 131 ; 141) sans interférer avec les caisses (3).

6. Système de stockage selon la revendication 5, dans lequel le système convoyeur (2) comporte des moyens pour transporter, de préférence soulever, les caisses (3) pour déplacer les caisses (3) ou une colonne (39) de caisses (3) empilées le long des éléments de support (1, 30 ; 101 ; 111 ; 121 ; 131 ; 141).

7. Système de stockage selon la revendication 5 ou 6, dans lequel le système convoyeur (2) peut être déplacé par un entraînement (25) ou
comprend en outre un entraînement (25) pour déplacer le système convoyeur (2) le long de l'allongement des éléments de support (1, 30).

8. Système de stockage selon l'une quelconque des revendications précédentes, dans lequel le système de stockage (51) comprend au moins deux éléments de support (1, 30) disposés de manière consécutive dans une direction longitudinale le long de l'allongement des éléments de support (1, 30).

9. Système de stockage selon l'une quelconque des revendications précédentes, dans lequel les caisses (3) sont empilées dans au moins une colonne (39) de caisses empilées (3), et/ou
dans lequel les caisses (3) ou les colonnes (39) de caisses empilées (3) sont disposées de manière adjacente sur l'au moins un élément de support (1, 30).

10. Système de stockage selon l'une quelconque des revendications 5 à 9, dans la mesure où elles dépendent de la revendication 5,
le système convoyeur (2) comprenant des roues (21) ou des moyens de rotation.

11. Système de stockage selon la revendication 10, dans lequel le système convoyeur (2) est positionné par un système d'observation (56),
le système d'observation (56) comprend au moins un laser et/ou au moins une caméra.

12. Système de stockage selon l'une quelconque des revendications précédentes, dans lequel les caisses (3) sont adaptées pour permettre l'élevage d'insectes ou de larves d'insectes ou de vers.

13. Unité d'élevage (81) pour l'élevage d'insectes ou de larves d'insectes ou de vers comprenant une salle d'élevage (83) et un système de stockage (71 ; 76) selon l'une quelconque des revendications 1 à 12.
